# EUROPÄISCHE PATENTANMELDUNG

(11) **EP 1 138 386 A1**
(43) Veröffentlichungstag der Anmeldung: **04.10.2001**
(21) Anmeldenummer: 00106670.3
(22) Anmeldetag: 29.03.2000
(51) Int. Cl.: B01J 29/04, C07D 301/12, C07C 249/04

(54) **Verfahren zur Herstellung eines Titansilicalitformkörpers**

(71) Anmelder: Degussa AG, 40474 Düsseldorf (DE)
(72) Erfinder: Hasenzahl, Steffen, Dr., 63477 Maintal (DE); Jantke, Ralf, 63776 Mömbris (DE)
(74) Vertreter: Sternagel, Fleischer, Godemeyer & Partner Patentanwälte

(57) **Zusammenfassung**

Die vorliegende Erfindung betrifft ein erfahren zur Herstellung eines Titansilicalitformkörpers durch:
a) Ausbilden eines Synthesegels enthaltend eine SiO₂-Quelle, eine TiO₂-Quelle, eine Templatverbindung und Wasser,
b) Kristallisieren des Synthesegels unter hydrothermalen Bedingungen,
c) Trocknen des Titansilicalits aus Schritt b) bei einer Temperatur unterhalb der Zersetzungstemperatur der Templatverbindung,
d) Ausbilden einer verformbaren Masse enthaltend das Produkt aus Schritt c), ein Bindemittel und ein Anteigungsmittel,
e) Verformen der Masse aus Schritt d) zu einem Grünkörper,
f) eventuell Trocknen und
g) Calcinieren des Grünkörpers sowie
einen nach diesem Verfahren erhältlichen Titansilicalitformkörper und die Verwendung diese Formkörpers als Katalysator in Epoxidierungs- oder Ammoximierungsreaktionen.

## Beschreibung

Die vorliegende Erfindung betrifft ein Verfahren zur Herstellung eines Titansilicalitformkörpers, einen Titansilicalitformkörper erhältlich nach dem oben beschriebenen Verfahren und Verfahren zur Epoxidierung von Olefinen sowie zur Ammoximierung von Ketonen unter Verwendung dieses Titansilicalitformkörpers.

Aus US-A-4,410,501 ist ein Verfahren zur Herstellung von Titansilicalit wie auch die Verwendung des so hergestellten Titansilicalits als Katalysator in einer Reihe von Reaktionen, u. a. Oxidationsreaktionen, bekannt. Die Herstellung des Titansilicalits erfolgt durch Ausbildung eines Synthesegels, ausgehend von einer hydrolysierbaren Siliciumverbindung wie z. B. Tetraethylorthosilicat und einer hydrolysierbaren Titanverbindung durch Zugabe von Tetra-n-propylammoniumhydroxid, Hydrolysieren und Kristallisieren dieser Reaktionsmischung. Nach Beendigung der Kristallisation werden die Kristalle durch Filtration abgetrennt, gewaschen, getrocknet und schließlich 6 h lang bei 550°C calciniert.

In DE-A 197 31 672 ist ein Verfahren zur Herstellung von Titansilicalitgranulaten beschrieben, wonach ein Synthesegel, das eine SiO₂-Quelle, eine TiO₂-Quelle Tetra-n-propylammoniumionen enthaltende Verbindung, eine Base und Wasser enthält unter Hydrothermalen Bedingungen kristallisiert wird und danach die dabei entstehende Titansilicalitsuspension ohne vorherige Abtrennung einer Sprühtrocknung oder einer Wirbelschichtsprühgranulationstrocknung unterzogen wird, und danach das gebildete Titansilicalitgranulat bei einer Temperatur von 400°C bis 1.000°C, vorzugsweise von 500°C bis 750°C calciniert wird.

Insbesondere bei Verwendung des Katalysators in einem Festbett bei der Epoxidierung von Olefinen bzw. der Ammoximierung von Ketonen ist es erstrebenswert, größere Formkörper auszubilden, die eine ausreichende Härte und Abriebfestigkeit aufweisen, damit die physikalische Integrität der Katalysatorformkörper auch nach längerem Betrieb bestehen bleibt. Insbesondere soll die Bildung von Feinkorn, das mit dem Reaktionsprodukt ausgetragen wird, was zum einen zu Katalysatorverlusten führt und zum anderen eine aufwendigere Auftrennung der Reaktionsprodukte notwendig macht, verringert bzw. vermieden werden.

Aus DE-A 196 23 611 ist ein Verfahren zur Herstellung von Titansilicalitformkörpern bekannt, nach dem zuerst ein Synthesegel durch Hydrolyse einer Siliciumquelle, einer Titanquelle und Tetrapropylammoniumhydroxid hergestellt wird, dieses Synthesegel unter hydrothermalen Bedingungen kristallisiet wird, der entstandene Feststoff z. B. durch Zentrifugation abgetrennt wird, getrocknet und anschließend bei 550°C calciniert wird. Dieses calcinierte Titansilicalitpulver wird dann anschließend durch Zugabe von Wasser, Bindemittel wie z. B. Kieselsol und Plastifizierungsmittel wie Methylcellulose zu einer plastischen Masse weiterverarbeitet, die zu Strängen extrudiert wird, diese Stränge getrocknet und noch einmal bei 500°C 5 h lang calciniert werden. Die Stränge werden dann zu Granulat oder Split mit einer Korngröße zwischen 1 und 10 mm zerkleinert.

WO 98/55229 offenbart ein vergleichbares Verfahren bei dem die verformbare Masse durch Zugabe einer Mischung von Wasser und Alkohol als Anteigungsmittel hergestellt wird.

Allen diesen aus dem Stand der Technik bekannten Verfahren ist gemeinsam, daß der Titansilicalit, der nach der hydrothermalen Kristallisation entsteht, vor weiteren Formgebungsschritten calciniert wird und nach der erfolgten Formgebung der resultierende Formkörper ein zweites Mal calciniert wird. Aufgabe der vorliegenden Erfindung ist es daher, ein Verfahren zur Herstellung von Titansilicalitformkörpern bereitzustellen, das im Vergleich zu den Verfahren des Standes der Technik, effizienter und kostengünstiger ist, ohne dadurch die erwünschten Eigenschaften der Titansilicalitformkörper als Katalysatoren in Oxidierungsreaktionen von Olefinen bzw. bei der Ammoximierung von Ketonen nachteilig zu beeinflussen. Eine weitere Aufgabe der vorliegenden Erfindung ist einen Titansilicalitformkörper mit verbesserten Eigenschaften bereitzustellen.

Diese Aufgabe wird durch ein Verfahren zur Herstellung eines Titansilicalitformkörpers durch
a) Ausbilden eines Synthesegels enthaltend eine SiO₂-Quelle, eine TiO₂-Quelle, eine Templatverbindung und Wasser,
b) Kristallisieren des Synthesegels unter hydrothermalen Bedingungen,
c) Trocknen des Titansilicalits aus Schritt b) bei einer Temperatur unterhalb der Zersetzungstemperatur der Templatverbindung,
d) Ausbilden einer verformbaren Masse enthaltend das Produkt aus Schritt c), ein Bindemittel und ein Anteigungsmittel,
e) Verformen der Masse aus Schritt d) zu einem Grünkörper,
f) eventuell Trocknen und
g) Calcinieren des Grünkörpers,
sowie durch einen nach diesem Verfahren erhältlichen Titansilicalitformkörper gelöst.

Im Gegensatz zu den Verfahren nach dem Stand der Technik wird der Festkörper aus dem Kristallisationsschritt der Titansilicalitsynthese bei einer Temperatur unterhalb der Zersetzungstemperatur der Templatverbindung, vorzugsweise bei einer Temperatur im Bereich von 50 bis 400°C getrocknet und dann das getrocknete, aber noch nicht calcinierte Produkt einem Formgebungsschritt unterworfen.

Das Calcinieren ist im allgemeinen ein sehr energieaufwendiger Schritt, da es bei hohen Temperaturen im Bereich von 400°C bis 1.000°C, insbesondere von 500°C bis 750°C für mehrere Stunden, in der Regel 3 bis 15 h, vorzugsweise 3 bis 6 h durchgeführt wird. Da das erfindungsgemäße Verfahren lediglich einen Calcinierungsschritt nach Ausbildung des Grünkörpers vorsieht, ist es im Vergleich zum Stand der Technik wesentlich weniger energieintensiv und somit deutlich kostengünstiger. Darüber hinaus wurde überraschenderweise gefunden, daß die erwünschten Eigenschaften des so hergestellten Katalysatorformkörpers wie katalytische Aktivität und Selektivität für Olefinoxid in einer Epoxidierungsreaktion von Olefin nicht beeinträchtigt werden und im Gegenteil die mechanischen Eigenschaften wie z. B. Bruchhärten noch verbessert werden können. Somit ist ein Titansilicalitformkörper nach dem erfindungsgemäßen Verfahren erhältlich, der eine verbesserte Bruchhärte aufweist und somit insbesondere als Katalysatorfüllung für Festbettreaktoren zur Verwendung bei der Epoxidierung von Olefinen geeignet ist.

Gemäß einer Ausführungsform des erfindungsgemäßen Verfahrens wird die Kristallsuspension, die nach der hydrothermalen Kristallisation entsteht, einem Fest-Flüssig-Trennverfahren wie z. B. Zentrifugation oder Filtration unterworfen, um die entstandenen Titansilicalitkristallite abzutrennen. Der abgetrennte Feststoff wird dann vorteilhafterweise mehrmals gewaschen und bei einer Temperatur unterhalb der Zersetzungstemperatur der Templatverbindung getrocknet.

Insbesondere bei Titansilicalitkristallsuspensionen mit Kristallitagglomeraten kleiner Korngrößen können allerdings bei der Filtration Schwierigkeiten auftreten. So sind dann z. B. Filter mit geringen Porengrößen zu verwenden, die insbesondere zu längeren Filtrationszeiten und hohen Druckverlusten während der Filtration führen, was die Filtration uneffizient macht. Dieser Nachteil kann zum einen dadurch umgangen werden, daß die Kristallsuspension neutralisiert wird. Die Kristallsuspension, wie sie nach Beendigung der Kristallisation anfällt, ist aufgrund des Überschusses an basischer Templatverbindung alkalisch und weist in der Regel einen pH von > 12 auf. Wird der pH-Wert der Suspension auf einen Wert 7 bis 10, vorzugsweise 7 bis 8,5 herabgesetzt, beobachtet man eine stärkere Agglomeration der Primärkristallite. Dadurch wird die Filtrierbarkeit der Suspension stark verbessert, so daß die Abtrennung mit standardmäßigen Membranfiltern ohne Durchschlagen des Produkts und mit üblichen Filtrationszeiten durchgeführt werden kann. Somit kann mit dieser bevorzugten Ausführungsform die Effizienz des erfindungsgemäßen Verfahrens noch weiter gesteigert werden.

In einer alternativen Ausführungsform, die sich insbesondere für Kristallsuspensionen eignet, die schwer filtrierbar sind, wird die Kristallsuspension direkt ohne vorherige Abtrennung des Festkörpers bei einer Temperatur unterhalb des Zersetzungspunktes der Templatverbindung getrocknet. In einer Ausführungsform der Erfindung erfolgt die Trocknung mittels Sprühtrocknung. Die so hergestellten Titansilicalitgranulate sind aus Titansilicalitkristallen, Siliciumdioxid und Titandioxid zusammengesetzt, wobei der Gehalt an Siliciumdioxid zwischen 1 und 10 Gew.-% und der Gehalt an Titandioxid zwischen 0,05 und 1 Gew.-% liegen kann. Die mittels Sprühtrocknung hergestellten Granulate können einen Durchmesser zwischen 5 bis 300 *µ*m aufweisen und teilweise hohl sein.

Alternativ kann die Kristallitsuspension auch mittels Wirbelschichtsprühgranulationstrocknung getrocknet werden.

In einer bevorzugten Ausführungsform kann bei der direkten Trocknung der Kristallsuspension aus dem Hydrothermalschritt der Feststoffgehalt vor der Sprühtrocknung oder der Wirbelschichtsprühgranulationstrocknung erhöht werden, so daß der Feststoffgehalt der Titansilicalitsuspension vorzugsweise mindestens 50 Gew.-% beträgt. Dazu kann man beispielsweise die nach der Kristallisation erhaltene Titansilicalitsuspension teilen und aus einem Teil der Suspension der Feststoff durch kuchenbildende Filtration, Zentrifugation oder andere geeignete Verfahren abtrennen, die Filterkuchen oder das Sediment kann man anschließend gegebenenfalls nach einem Waschschritt in dem verbliebenen Teil der Titansilicalit-1-Suspension suspendieren. Um die Abtrennung des Feststoffs zu erleichtern, kann man der Titansilicalitsuspension geeignete Flockungshilfsmittel zusetzen. Den Feststoffgehalt der Titansilicalitsuspension kann man auch durch Eindampfung, vorzugsweise unter vermindertem Druck, oder Cross-flow-Filtration erhöhen. Die Aufkonzentration der Titansilicalitsuspension vor dem Trockenschritt ist besonders bevorzugt, weil dadurch der Energieaufwand bezogen auf die Masse getrockneter Titansilicalit verringert werden kann.

Die Sprühtrocknung bzw. Wirbelschichtsprühgranulationstrocknung, die gemäß der vorliegenden Erfindung vorteilhafterweise eingesetzt wird, ist detailliert in DE 197 31 672, insbesondere in den darin angegebenen Beispielen beschrieben.

Eine alternative Ausführungsform betrifft ein Verfahren zur Herstellung eines Titansilicalitformkörpers durch:
a) Ausbilden eines Synthesegels enthaltend eine SiO₂-Quelle, eine TiO₂-Quelle, eine Templatverbindung und Wasser,
b) Kristallisieren des Synthesegels unter hydrothermalen Bedingungen,
c) Aufkonzentrieren der Kristallsuspension aus Schritt b),
d) Ausbilden einer verformbaren Masse enthaltend das Produkt aus Schritt c) und ein Bindemittel,
e) Verformen der Masse aus Schritt d) zu einem Grünkörper,
f) eventuell Trocknen und
g) Calcinieren des Grünkörpers.

Die Aufkonzentrierung der Suspension in Schritt c) kann dabei wie bei der vorgehend beschriebenen Ausführungsform erfolgen. Der Feststoffgehalt der Titansilicalitsuspension nach der Aufkonzentrierung beträgt vorzugsweise 10 - 50 Gew.-%. Diese Ausführungsform hat den zusätzlichen Vorteil, daß auch der Energieaufwand und die damit verbundenen Kosten für die Abtrennung und Trocknung des Titansilicalits eingespart werden können.

Bei der Herstellung des Synthesegels vor der Kristallisation des Titansilicalits wird eine SiO₂-Quelle, eine TiO₂-Quelle, eine Templatverbindung sowie Wasser zusammengegeben. Insbesondere eignen sich hydrolysierbare Siliciumverbindungen und hydrolysierbare Titanverbindungen als Ausgangsverbindungen, die dann in Gegenwart von Wasser hydrolysiert werden. Geeignete hydrolysierbare Silicium- bzw. Titanverbindungen sind die Tetraalkylorthosilicate bzw. Tetraalkylorthotitanate wobei Alkyl vorzugsweise ausgewählt ist aus der Gruppe bestehend aus Methyl, Ethyl, Propyl oder Butyl. Die am meisten bevorzugten Ausgangsverbindungen sind Tetraethylorthosilicat und Tetraethylorthotitanat. Unter Templatverbindungen versteht man Verbindungen, die durch Aufnahme in das Kristallgitter des Produkts während der Kristallisation die Kristallstruktur bestimmen. Bevorzugte Templatverbindungen sind quartäre Ammoniumverbindungen wie Tetraalkylammoniumverbindungen, insbesondere Tetraalkylammoniumhydroxid wie Tetra-n-propylammonium-hydroxid zur Herstellung von Titansilicalit-1 (MFI-Struktur), Tetra-n-butylammoniumhydroxid zur Herstellung von Titansilicalit-2 (MEL-Struktur) und Tetraethylammoniumhydroxid zur Herstellung von Titan-β-Zeolith (DEA-Kristallstruktur).

Der für die Synthese erforderliche pH-Wert des Synthesegels von > 9, bevorzugt > 11 stellt sich durch die basisch reagierende quartäre Ammoniumverbindung, die als Templat eingesetzt wird, ein. Die Temperatur, bei der das Synthesegel hergestellt wird, kann in weiten Grenzen variiert werden, bevorzugt ist allerdings das Gemisch aus Siliciumquelle, Titanquelle auf eine Temperatur im Bereich von 0°C bis 10°C bis vorzugsweise 0°C bis 5°C, besonders bevorzugt 1°C abzukühlen und dann die Templatverbindung in wäßriger Lösung, die auf die gleiche Temperatur abgekühlt ist, zuzugeben.

In einer weiteren Ausführungsform der vorliegenden Erfindung wird bei der Verwendung von Tetraalkylorthosilicaten und Tetraalkylorthotitanaten als Silicium- bzw. Titanquelle das Synthesegel auf eine Temperatur von 75°C bis 95°C für eine Zeitdauer von 120 bis 200 min erwärmt und der entstehende Alkohol als Wasserazeotrop abdestilliert, um die Hydrolyse der hydrolysierbaren Titan- und Siliciumverbindungen zu unterstützen. Das Synthesegel wird anschließend gegebenenfalls nach einer zusätzlichen Reifezeit bei einer Temperatur von 150°C bis 220°C, vorzugsweise 170°C bis 190°C unter autogenem Druck kristallisiert. Unter den vorgegebenen Bedingungen beträgt die Kristallisationszeit in der Regel weniger als 3 Tage, vorzugsweise weniger als 24 h.

Ebenfalls gut geeignete Ausgangsverbindungen sind Silicium-Titan-Mischoxide, die beispielsweise durch Flammenhydrolyse eines Gemisches von SiCl₄ und TiCl₄ hergestellt werden können. Diese können in einer geeigneten Templat- und Base-enthaltenden Lösung dispergiert und nach einem evtl. Alterungsschritt bzw. der Zugabe von Impfkristallen wie oben beschrieben kristallisiert werden.

Nach der Trocknung wird der Titansilicalitfestkörper bzw. die aufkonzentrierte Titansilicalitsuspension mit einem oder mehreren Bindemittel, gegebenenfalls mit Formgebungshilfsmittel und gegebenenfalls einem Anteigungsmittel zu einer verformbaren Masse verarbeitet. Als Bindemittel eignen sich im Prinzip alle für derartige Zwecke bekannten Stoffe. Bevorzugt werden Oxide des Siliciums, Aluminiums, Bors, Phosphors, Titans, Zirkoniums und/oder Magnesiums bzw. entsprechende Vorstufen verwendet. Besonders bevorzugte Bindemittel sind Siliciumdioxide, Aluminiumoxide, Titanoxide und Tonmineralien sowie Mischungen hiervon. Beispiele für Siliciumdioxide bzw. Siliciumdioxid-Vorstufen sind gefällte oder pyrogene Kieselsäuren, Kieselsole, Tetraalkoxysilane, teilkondensierte Kieselsäureester und Polysiloxane, für Aluminiumoxid (Pseudo-) Boehmit, für Titandioxid Anatas oder Brookit und für Tonmineralien Montmorillonite, Kaoline, Bentonite und Sepiolithe.

Die Bindemittel können als Pulver oder in Form von Solen, Suspensionen oder Lösungen verwendet werden. Die Menge an Bindemittel beträgt im allgemeinen 1 - 99 Gew.-%, vorzugsweise 5 - 60 Gew.-% und besonders 10 - 40 Gew.-% bezogen auf den Feststoffgehalt der verformbaren Masse.

Als Formgebungshilfsmittel können Substanzen zugesetzt werden, die in erster Linie die Bildung einer plastischen Masse während des Knetverformens und Trockenschritts und darüber hinaus die mechanische Stabilität des Formkörpers beim Verformen und Trocknen begünstigen. Diese Substanzen werden vorzugsweise beim Calcinieren des Grünkörpers entfernt. Bevorzugt sind organische viskositätssteigernde Substanzen, wie z. B. Cellulose, Stärke, Acrylat, Polyacrylat, Polymethacrylat, Polyvinylalkohol, Polyvinylpyrolidon, Polyisobuten und Polytetrahydrofuran, Polyglykolether, Fettsäureverbindungen und/oder Wachsemulsionen.

Als weitere Zusatzstoffe können Basen oder Säuren zugesetzt werden. Geeignet sind beispielsweise Ammoniak, Amine oder quarternäre Ammoniumsalze sowie Carbonsäuren, wie z. B. Ameisen-, Essig- oder Propionsäure. Selbstverständlich können auch Gemische aus zwei oder mehr der oben genannten Zusatzstoffe verwendet werden.

Als weitere Zusatzstoffe können Porenbildner, wie Polyalkohole, Fructose, Pentaerythrit, Cellulose oder Sägemehl eingesetzt werden. Diese brennen bei der Calcinierung heraus und hinterlassen zusätzliche Meso- und Makroporen im Formkörper.

Formgebungshilfsmittel werden in dem erfindungsgemäßen Verfahren in einer Menge von 0 bis 40 Gew.-%, vorzugsweise 1 - 10 Gew.-%, bezogen auf den Festkörpergehalt der verformbaren Masse, eingesetzt.

Als Anteigungsmittel wird vorzugsweise in dem erfindungsgemäßen Verfahren ein wäßriges Medium verwendet, das gegebenenfalls ein wassermischbares organisches Lösungsmittel enthält. Wenn als Bindemittel ein Metallsäureester wie z. B. Alkylorthosilicat oder Alkylorthotitanat verwendet wird ist es bevorzugt, als Anteigungsmittel eine Mischung aus Wasser und einem Alkohol, der der Alkoholkomponente des Metallsäureesters entspricht, zu verwenden. Bei der Verwendung eines Gemisches aus Alkohol und Wasser als Anteigungsmittel beträgt der Alkoholgehalt dieser Mischung im allgemeinen 1 bis 80 Gew.-%, vorzugsweise 5 bis 70 Gew.-% und insbesondere bevorzugt 10 bis 60 Gew.-%, jeweils bezogen auf das Gesamtgewicht der Mischung.

Das Anteigungsmittel wird in Mengen eingesetzt, so daß eine plastische extrudierbare Masse entsteht, vorzugsweise in einer Menge, so daß der Festkörpergehalt der verformbaren Masse 10 - 90 Gew.-%, vorzugsweise 60 - 80 Gew.-% beträgt.

Der verformbaren Masse können außerdem beliebige weitere Komponenten, wie z. B. weitere Zeolithe, pyrogene bzw. gefällte Oxide und Metalle, sowie zur Verbesserung der mechanischen Stabilität, anorganische Fasern, wie z. B. Al-Si-Fasern und/oder Ca-Si-Fasern zugegeben werden.

Die Zugabenreihenfolge der Bestandteile der verformbaren Masse hängt von einer Reihe von Faktoren ab und muß im Einzelfall ausgearbeitet werden Es ist sowohl möglich, zuerst zu dem Titansilicalitfeststoff das Bindemittel zuzugeben, anschließend die gegebenenfalls verwendeten Formgebungshilfsmittel und zum Schluß das Anteigungsmittel zuzugeben. Nach der Zugabe des Bindemittels und gegebenenfalls des Formgebungshilfsmittels wird die in der Regel noch pulverförmige Masse im Kneter, Extruder oder Mischer homogenisiert und dann das Anteigungsmittel zugesetzt. Die so erhaltene Mischung wird solange gemischt, bis eine verstrangbare oder extrudierbare plastische Masse entstanden ist. Dabei wird in der Regel bei Temperaturen im Bereich von 10°C bis zum Siedepunkt des Anteigungsmittels und Normaldruck, Unterdruck oder leichtem überatmosphärischem Druck gearbeitet.

Bei der Ausführungsform, die von der aufkonzentrierten Titansilicalitsuspension ausgeht, wird vorzugsweise das Bindemittel und gegebenenfalls Formgebungshilfsmittel der Titansilicalitsuspension zugegeben und falls erforderlich, um eine knetbare Masse zu ergeben, zusätzlich ein Anteigungsmittel zugegeben.

Es können für die Mischung und Verformung alle bekannten Misch- und Verformungsvorrichtungen bzw. -verfahren verwendet werden. Derartige bekannte Verformungsvorrichtungen werden beispielsweise in Ullmann's Enzyklopädie der Technischen Chemie, 4. Auflage, Band 2, Seite 295 ff., 1972 beschrieben. Bevorzugt werden Ein- und Zweischneckenextruder oder eine Strangpresse eingesetzt. Dabei können zahlreiche bekannte Geometrien, wie z. B. Vollzylinder, Hohlzylinder, Sterne usw. hergestellt werden. Möglich ist jedoch auch die Herstellung von Wabenkörpern.

Die so erhaltenen Grünkörper, deren Durchmesser zwischen 1 und 10 mm liegt können anschließend bei Raumtemperatur oder erhöhten Temperaturen, ggf. in Gegenwart von Wasserdampf, getrocknet werden.

Anschließend werden die Formkörper in Gegenwart von Luft, Inertgas oder Sauerstoff bei Temperaturen bis zu 1.100°C calciniert. Der zeitliche Verlauf der Temperatur, d. h. Aufheizrate, Haltezeiten bei Zwischen- und Endtemperatur, sowie Abkühlrate müssen dabei im Einzelfall optimiert werden. Die Kalzinierung kann zur Entfernung des Templats und der Zusatzstoffe aus dem Grünkörper dienen und beeinflußt in entscheidender Weise die mechanische Stabilität, das Porenvolumen oder die spezifische Oberfläche.

Die erhaltenen Stränge bzw. Extrudate können zerkleinert werden. Sie werden dabei vorzugsweise zu einem Granulat oder Splitt mit einem Partikeldurchmesser von 0,1 bis 5 mm, insbesondere von 0,5 bis 2 mm zerkleinert.

Zur Verdeutlichung der vorliegenden Erfindung werden die folgenden Beispiele angegeben:

### Bezugsbeispiel 1

### Herstellung einer Titansilicalitsuspension

In einem mit Stickstoff inertisierten 10-1-Autoklaven wurden Tetraethylorthosilicat, Tetraethylorthotitanat und Tetra-n-propylammoniumhydroxid in wäßriger Lösung zusammengegeben und hydrolysiert. Die Mengen wurden dabei so gewählt, daß das molare Verhältnis Si/Ti = 35 war, das molare Verhältnis von N/Si = 0,17 und das molare Verhältnis von H₂O/Si = 27 betrug. Nach Beendigung der Hydrolyse und Abdestillieren des gebildeten Alkohols, der durch das gleiche Volumen Wasser ersetzt wurde, wurde das Synthesegel 3 h lang bei 175°C unter Hydrothermalbedingungen kristallisiert. Die so erhaltene Titansilicalitsuspension wurde gemäß der folgenden Beispiele und Vergleichsbeispiele in unterschiedlicher Weise weiterverarbeitet.

### Beispiel 1

Die Titansilicalitsuspension des Bezugsbeispiels 1 wurde sprühgetrocknet. In einem 2,5-1-Kneter mit Austragsschnecke wurden 750 g des so getrockneten Titansilicalits, 20 g einer Methylhydroxycellulose, 200 g Aluminiumoxid eingewogen, 10 min gemischt und anschließend 30 g Eisessig zugegeben. Danach wurden innerhalb von 20 min soviel Wasser zugegeben, bis sich eine kompakte Masse bildete, die weitere 45 min geknetet wurde. Für die Extrusion wurde eine kernprogressive Schnecke verwendet. Bei der Herstellung der 4-mm-Vollzylinder kam ein Mundstück mit einer entsprechenden Bohrung zum Einsatz. Die extrudierten Formkörper wurden mit einem Gebläse angetrocknet, anschließend über Nacht an Luft stehengelassen und schließlich bei 550°C 5 h lang calciniert.

Die Seitenbruchfestigkeit des so hergestellten Formkörpers mittels eines Tablettenbruchfestigkeits-Testers (TBH 28 Erweka) bestimmt. Der Mittelwert von insgesamt 20 Messungen betrug 23 N.

### Beispiel 2

Entsprechend der Vorgehensweise in Beispiel 1 wurden 823 g sprühgetrockneter Titansilicalit, 157 g teilhydrolysiertes Tetraethylorthosilicat (Ester 40 der Fa. Wacker, 40 Gew.-% SiO₂) und 20 g Methylhydroxycellulose vorgelegt und vermischt. Danach wurde solange Wasser zugesetzt, bis sich eine knetbare, verformbare Masse gebildet hatte. Diese Masse wurde weitere 45 min lang geknetet und dann analog zu Beispiel 1 extrudiert. Die extrudierten Formkörper wurden wie in Beispiel 1 angegeben getrocknet und 1 h lang bei 550°C calciniert.

Der calcinierte Titansilicalitformkörper wies eine Seitenbruchfestigkeit von 58 N auf.

### Beispiel 3

Gemäß der Vorgehensweise in Beispiel 1 wurden 780 g des sprühgetrockneten Titansilicalits, 200 g eines Tonminerals der Zusammensetzung Al₂O₃, 18,55 Gew.-%, SiO₂ 74,98 Gew.-%, MgO 3,36 Gew.-%, BaO 1,42 Gew.-%, FeO 2,3 0,25 Gew.-% und TiO₂ 0,18 Gew.-% und 20 g Methylhydroxycellulose vorgelegt und miteinander vermischt. Danach wurden innerhalb von 20 min soviel Wasser zugegeben bis sich eine kompakte Masse bildete, die weitere 45 min geknetet wurde. Die Extrusion wurde analog zu Beispiel 1 durchgeführt und die resultierenden extrudierten Formkörper wurden mit einem Gebläse angetrocknet, anschließend über Nacht an Luft stehengelassen und schließlich bei 550°C 1 h lang getrocknet.

Daraus resultierte eine Seitenbruchfestigkeit von 19 N für den calcinierten Titansilicalitformkörper.

### Vergleichsbeispiele 1 bis 3

Die Beispiele 1 bis 3 wurden wiederholt mit dem Unterschied, daß der getrocknete Titansilicalitfestkörper vor der Weiterverarbeitung 5 h bei 550°C calciniert wurde. Für den calcinierten Formkörper nach Vergleichsbeispiel 1 ergab sich eine Seitenbruchfestigkeit von 17 N. Die Seitenbruchfestigkeit des calcinierten Formkörpers nach Vergleichsbeispiel 3 betrug 14 N. Die nach Vergleichsbeispiel 2 hergestellte Masse war nicht extrudierbar, so daß hier keine Formkörper hergestellt werden konnten.

### Beispiel 4

Die aus dem Bezugsbeispiel 1 resultierende Titansilicalitsuspension wurde durch Abdestillieren des Lösungsmittels (vor allem Wasser) auf einen Feststoffgehalt von 54 Gew.-% aufkonzentriert. 94,1 g dieser Suspension und 2,0 g Methylhydroxycellulose wurden in einem 0,25-l-Kneter vorgelegt und langsam 38,0 g sprühgetrocknetes Titansilicalitpulver entsprechend des Beispiels 1 zugegeben. Nach weiterer Zugabe von 1,5 g Ammoniumacetat und 20 g des Tonminerals gemäß Beispiel 3 und 13 g Wasser bildete sich eine weiche und geschmeidige Masse, die weitere zwei Stunden geknetet wurde. Die gut extrudierbare Masse wurde analog zu Beispiel 1 extrudiert und die resultierenden Formkörper mit einem Gebläse angetrocknet über Nacht stehengelassen und bei 550°C 1 h lang in einem Kammerofen calciniert.

Der resultierende calcinierte Formkörper wies eine Seitenbruchfestigkeit von 44 N auf.

Der Vergleich zwischen den Beispielen gemäß der vorliegenden Erfindung und den Vergleichsbeispielen zeigt, daß zum einen geeignete Katalysatorformkörper mit dem erfindungsgemäßen Verfahren hergestellt werden können, wobei lediglich ein Calcinierungsschritt nach Ausbildung der Grünkörper notwendig ist und somit im Vergleich zum Stand der Technik weniger Energie zur Herstellung eines Katalysatorformkörpers benötigt wird. Darüber hinaus weisen diese Formkörper im Vergleich zu den Formkörpern, die ausgehend von calciniertem Titansilicalitpulver hergestellt wurden, eine deutlich verbesserte Bruchhärte gemessen als Seitenbruchfestigkeit auf.

Beispiel 4 zeigt eine besonders bevorzugte Ausführungsform, nach der nach Aufkonzentrieren der aus dem Hydrothermalschritt erhaltenen Titansilicalitsuspension diese direkt in dem Formgebungsschritt weiterverwendet wird. Diese Verfahrensvariante ist besonders kostengünstig, da hier im Vergleich zum Stand der Technik sich nicht nur ein Calcinierungsschritt, sondern auch das vorherige Abtrennen und Trocknen des Titansilicalits aus der Hydrothermalsynthese erübrigt. Überraschenderweise konnte bei dieser Verfahrensvariante die Bruchhärte der resultierenden Formkörper noch weiter gesteigert werden.

## Patentansprüche

1. Verfahren zur Herstellung eines Titansilicalitformkörpers durch:
a) Ausbilden eines Synthesegels enthaltend eine SiO₂-Quelle, eine TiO₂-Quelle, eine Templatverbindung und Wasser,
b) Kristallisieren des Synthesegels unter hydrothermalen Bedingungen,
c) Trocknen des Titansilicalits aus Schritt b) bei einer Temperatur unterhalb der Zersetzungstemperatur der Templatverbindung,
d) Ausbilden einer verformbaren Masse enthaltend das Produkt aus Schritt c), ein Bindemittel und ein Anteigungsmittel,
e) Verformen der Masse aus Schritt d) zu einem Grünkörper,
f) eventuell Trocknen und
g) Calcinieren des Grünkörpers.

2. Verfahren nach Anspruch 1, wobei
die Kristallsuspension aus Schritt b) gegebenenfalls neutralisiert wird, mit einem Fest-Flüssig-Trennverfahren der Festkörper abgetrennt wird und dieser dann gemäß Schritt c) getrocknet wird.

3. Verfahren nach Anspruch 1, wobei
die Kristallsuspension aus Schritt b) ohne vorherige Abtrennung des Festkörpers direkt gemäß Schritt c) getrocknet wird.

4. Verfahren nach Anspruch 3, wobei
die Titansilicalitsuspension aus Schritt b) durch Abdestillieren flüchtiger Bestandteile und/oder durch Zugabe von nicht calciniertem Titansilicalit aufkonzentriert wird.

5. Verfahren nach Anspruch 4, wobei
der Feststoffgehalt der Titansilicalitsuspension vor dem Trockenschritt c) mindestens 50 Gew-% beträgt.

6. Verfahren nach einem der vorstehenden Ansprüche, wobei
als Anteigungsmittel ein wäßriges Medium verwendet wird, das gegebenenfalls ein wassermischbares organisches Lösungsmittel enthält.

7. Verfahren nach einem der vorstehenden Ansprüche, wobei
die Trockentemperatur in Schritt c) x-y °C beträgt.

8. Verfahren zur Herstellung eines Titansilicalitformkörpers durch:
a) Ausbilden eines Synthesegels enthaltend eine SiO₂-Quelle, eine TiO₂-Quelle, eine Templatverbindung und Wasser,
b) Kristallisieren des Synthesegels unter hydrothermalen Bedingungen,
c) Aufkonzentrieren der Kristallsuspension aus Schritt b),
d) Ausbilden einer verformbaren Masse enthaltend das Produkt aus Schritt c) und ein Bindemittel,
e) Verformen der Masse aus Schritt d) zu einem Grünkörper,
f) eventuell Trocknen und
g) Calcinieren des Grünkörpers

9. Verfahren nach Anspruch 8, wobei
der Feststoffgehalt der Titansilicalitsuspension nach der Aufkonzentrierung in Schritt c) x-y Gew-% beträgt.

10. Verfahren nach einem der vorstehenden Ansprüche, wobei
als Bindemittel Verbindungen des Siliciums, Aluminiums, Bors, Phosphors, Zirkoniums und/oder Titans verwendet werden.

11. Verfahren nach Anspruch 10, wobei
das Bindemittel ausgewählt ist aus Aluminiumoxid, Siliciumoxid, hydrolisierbaren Siliciumverbindungen und partiellen oder vollständigen Hydrolysaten hiervon, Borverbindungen, Phosphorverbindungen, Tonmineralien und Mischungen hiervon.

12. Verfahren nach einem der vorstehenden Ansprüche, wobei
der Grünkörper bei einer Temperatur von 400°C bis 1000°C, vorzugsweise von 500°C bis 750°C calciniert wird.

13. Titansilicalitformkörper erhältlich nach einem Verfahren gemäß einem der vorstehenden Ansprüche.

14. Verfahren zur Epoxidierung von Olefinen mit wäßrigem Wasserstoffperoxid in Gegenwart von Titansilicalitformkörpern nach Anspruch 13.

15. Verfahren zur Ammoximierung von Ketonen mit wässrigem Wasserstoffperoxid und Ammoniak in Gegenwart von Titansilicalitformkörpern nach Anspruch 13.
